(19) Europäisches Patentamt
European Patent Office
Office européen des brevets

(11) EP 3 416 938 B1

(12) **EUROPEAN PATENT SPECIFICATION**

(45) Date of publication and mention of the grant of the patent:
**16.10.2019 Bulletin 2019/42**

(21) Application number: **17702135.9**

(22) Date of filing: **02.02.2017**

(51) Int Cl.:
*C07C 67/03* (2006.01) *C07C 67/08* (2006.01)
*C07C 67/31* (2006.01) *C08G 65/26* (2006.01)

(86) International application number:
**PCT/EP2017/052255**

(87) International publication number:
**WO 2017/140510 (24.08.2017 Gazette 2017/34)**

(54) **ALKOXYLATED HYDROXYBENZOIC ACID ESTERS OR AMIDES**

ALKOXYLIERTE HYDROXYBENZOESÄUREESTER ODER AMIDE

ESTERS OU AMIDES ALCOXYLÉS D'ACIDE HYDROXYBENZOÏQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2016 EP 16156196**

(43) Date of publication of application:
**26.12.2018 Bulletin 2018/52**

(73) Proprietor: **Clariant International Ltd**
**4132 Muttenz (CH)**

(72) Inventors:
- **COHRS, Carsten**
  **60316 Frankfurt am Main (DE)**
- **DIEDERICHS, Jan**
  **84508 Burgkirchen (DE)**
- **LEINWEBER, Dirk**
  **65779 Kelkheim (DE)**
- **MUTCH, Kevin**
  **60385 Frankfurt (DE)**
- **ROMANSKI, Steffen**
  **46483 Wesel (DE)**
- **BATCHELOR, Stephen Norman**
  **Wirral**
  **Merseyside CH63 3JW (GB)**
- **BIRD, Jayne Michelle**
  **Wirral**
  **Merseyside CH63 3JW (GB)**

(74) Representative: **Paczkowski, Marcus**
**Clariant Produkte (Deutschland) GmbH**
**Patent & License Management Chemicals**
**Industriepark Höchst / G 860**
**65926 Frankfurt am Main (DE)**

(56) References cited:
**EP-A1- 0 974 575** **EP-A1- 2 703 347**
**WO-A1-2012/107419** **WO-A2-2005/043127**
**JP-A- 2009 256 214** **US-A- 4 548 744**

- **HUA ZHANG ET.AL.: "Highly efficient synthesis of monodisperse poly (ethylene glycols) and derivatives through macrocyclization of oligo(ethyleen glycols)", ANGEW. CHEM. INT. ED., vol. 54, 2015, pages 3763-3767, XP002760095,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 416 938 B1

## Description

The present invention concerns the field of alkoxylated hydroxybenzoic acid esters and derivatives thereof or alkoxylated hydroxybenzoic acid amides and derivatives thereof and a method for their preparation. The alkoxylated hydroxybenzoic acid esters or amides and their derivatives may advantageously be used as anti-redeposition agents in washing applications.

Anti-redeposition agents used in laundry detergents help to prevent soil from resettling on a fabric after it has been removed during washing. This can for example be achieved by dispersing the soil in the washing liquor.

The washing of soiled fabrics with a laundry detergent composition is essentially a two-step process. In the first stage the detergent must remove the soil from the fabric and suspend it in the washing liquor. In the second stage the detergent composition must prevent the soil and other insoluble materials from re-depositing on the cloth before the fabric is removed from the washing liquor or the rinse liquor. Polymers are known to aid both processes. For example, soil release polymers enhance soil removal from the fabric whilst anti-redeposition polymers prevent the removed soil from re-depositing on the fabric.

Examples of suitable anti-redeposition agents include fatty acid amides, fluorocarbon surfactants, complex phosphate esters, styrene maleic anhydride copolymers, and cellulosic derivatives such as hydroxyethyl cellulose, hydroxypropyl cellulose, and the like.

US 4,240,918 e.g. describes polymers having anti-soiling and anti-redeposition properties, for example hydrophilic polyurethanes, certain copolyesters and mixtures thereof.

US 4,548,744 discloses water-soluble ethoxylated amine oxides having clay soil removal/anti-redeposition properties.

However, many of the known anti-redeposition agents possess the disadvantage that their performance and whitening effect in washing or laundry applications are insufficient.

Therefore, the problem to be solved by the present invention is to provide new anti-redeposition agents that have favourable performance and lead to enhanced "whiteness" when used in washing or laundry applications.

Surprisingly it has been found that this problem can be solved by alkoxylates according to the following formula (I)

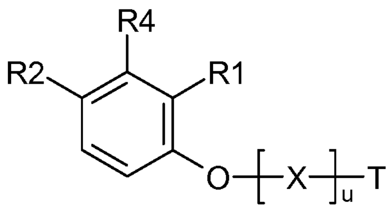

(I)

wherein

| | |
|---|---|
| X | is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy, |
| T | is selected from the group consisting of H, $C_1$-$C_4$ alkyl, $SO_3^-$, $CH_2$-$COO^-$, sulfosuccinate and $PO_3^{2-}$, preferably is selected from the group consisting of H and $CH_3$ and more preferably is H, |
| R1, R2 and R4 | are H, CO-NR8R3 or COO-$[X_1]_v$-R3, whereby two of the substituents R1, R2 and R4 are H and the third of these substituents is CO-NR8R3 or COO-$[X_1]_v$-R3 and preferably is COO-$[X_1]_v$-R3, |
| R8 | is H or a linear or branched alkyl group with 1 to 4 C-atoms, preferably H or $CH_3$ and more preferably H, |
| R3 | is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms, an aryl group with 6 to 10 carbon atoms, preferably phenyl, a group $(CH_2CH_2O)_m$aryl, wherein the aryl group comprises 6 to 10 carbon atoms and m, on a molar average, is a number of from 1 to 100, preferably of from 1 to 10, more preferably of from 1 to 3 and even more preferably of from 1 to 2, and preferably $(CH_2CH_2O)_m$aryl is $(CH_2CH_2O)_m$phenyl, or an aryl-substituted linear or branched $C_1$-$C_3$ alkyl group wherein the aryl group comprises 6 to 10 carbon atoms, preferably benzyl, but in the case, that R1 and R4 are H and R2 is COOR3 (i.e. COO-$[X_1]_v$-R3 wherein v = 0), R3 can only be an alkyl group, an alkenyl group or a group $(CH_2CH_2O)_m$aryl as defined above and in this case R3 preferably is an alkyl group or an alkenyl group as defined above, |
| $X_1$ | is selected from ethoxy and mixtures of ethoxy and propoxy groups, preferably is selected from ethoxy and mixtures of ethoxy and propoxy groups where the number of ethoxy groups in the mixtures is |

greater than the number of propoxy groups and more preferably $X_1$ is ethoxy,

u — on a molar average, is a number of from 3 to 100, preferably of from 5 to 60, more preferably of from 6 to 50, even more preferably of from 8 to 40 and particularly preferably of from 9 to 35, and

v — on a molar average, is a number of from 0 to 20, preferably of from 0 to 12, more preferably of from 0 to 10, even more preferably of from 0 to 8 and particularly preferably of from 0 to 7.

Therefore, a subject matter of the present invention is alkoxylates according to the following formula (I)

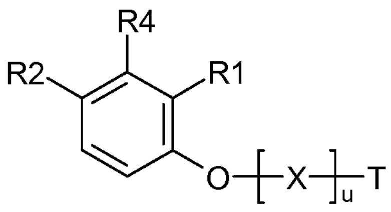

(I)

wherein

X — is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy,

T — is selected from the group consisting of H, $C_1$-$C_4$ alkyl, $SO_3^-$, $CH_2$-$COO^-$, sulfosuccinate and $PO_3^{2-}$, preferably is selected from the group consisting of H and $CH_3$ and more preferably is H,

R1, R2 and R4 — are H, CO-NR8R3 or COO-$[X_1]_v$-R3, whereby two of the substituents R1, R2 and R4 are H and the third of these substituents is CO-NR8R3 or COO-$[X_1]_v$-R3 and preferably is COO-$[X_1]_v$-R3,

R8 — is H or a linear or branched alkyl group with 1 to 4 C-atoms, preferably H or $CH_3$ and more preferably H,

R3 — is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms, an aryl group with 6 to 10 carbon atoms, preferably phenyl, a group $(CH_2CH_2O)_m$aryl, wherein the aryl group comprises 6 to 10 carbon atoms and m, on a molar average, is a number of from 1 to 100, preferably of from 1 to 10, more preferably of from 1 to 3 and even more preferably of from 1 to 2, and preferably $(CH_2CH_2O)_m$aryl is $(CH_2CH_2O)_m$phenyl, or an aryl-substituted linear or branched $C_1$-$C_3$ alkyl group wherein the aryl group comprises 6 to 10 carbon atoms, preferably benzyl, but in the case, that R1 and R4 are H and R2 is COOR3 (i.e. COO-$[X_1]_v$-R3 wherein v = 0), R3 can only be an alkyl group, an alkenyl group or a group $(CH_2CH_2O)_m$aryl as defined above and in this case R3 preferably is an alkyl group or an alkenyl group as defined above,

$X_1$ — is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably $X_1$ is ethoxy,

u — on a molar average, is a number of from 3 to 100, preferably of from 5 to 60, more preferably of from 6 to 50, even more preferably of from 8 to 40 and particularly preferably of from 9 to 35, and

v — on a molar average, is a number of from 0 to 20, preferably of from 0 to 12, more preferably of from 0 to 10, even more preferably of from 0 to 8 and particularly preferably of from 0 to 7.

The substituent T in formula (I) may have the meaning "sulfosuccinate". Sulfosuccinate has the structure

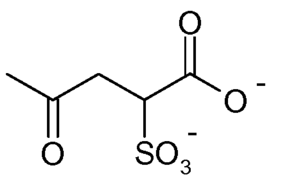

and/or

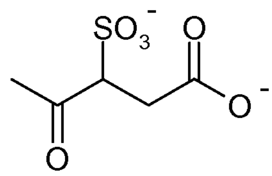

The group "ethoxy" e.g. mentioned in the definitions of "X" and "$X_1$" in formula (I) and also known as "ethyleneoxy" has the structure $-CH_2CH_2O-$.

The group "propoxy" e.g. mentioned in the definitions of "X" and "$X_1$" in formula (I) and also known as "propyleneoxy" has the structure

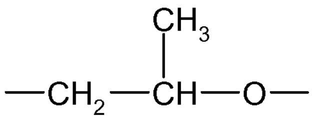 and/or 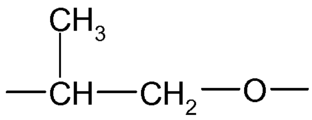

The substituent R3 in formula (I) may have the meaning "aryl-substituted linear or branched $C_1$-$C_3$ alkyl". The aryl-substituted linear or branched $C_1$-$C_3$ alkyl group denotes a linear or branched $C_1$-$C_3$ alkyl group which is substituted by an aryl group. The aryl group preferably comprises 6 to 10 carbon atoms and more preferably is phenyl. Particularly preferably, the substituent "aryl-substituted linear or branched $C_1$-$C_3$ alkyl" is benzyl $C_6H_5$-$CH_2$-.

The inventive alkoxylates according to formula (I) exhibit favourable performance as anti-redeposition agents in washing or laundry applications. They exhibit favourable performance as dispersants and in washing or laundry applications they lead to enhanced "whiteness". Furthermore, the inventive alkoxylates according to formula (I) exhibit favourable stability and in particular favourable hydrolytic stability. Preferably, inventive alkoxylates according to formula (I) furthermore exhibit favourable biodegradability.

Certain alkoxylated hydroxybenzoic acid esters are already disclosed in the prior art, for example, in JP 2009-256214 compounds such as

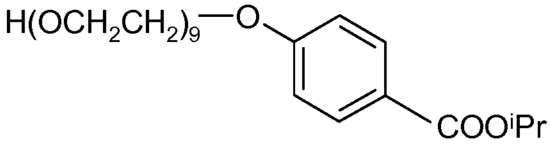

wherein "$^i$Pr" designates an iso-propyl residue -$CH(CH_3)_2$,
in US 5,480,761 compounds such as

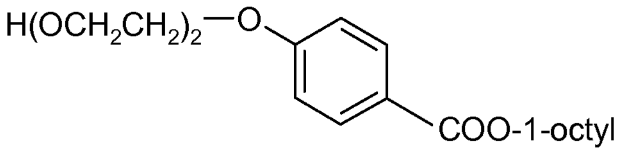

and in EP 2 703 347 compounds such as those of the following formulae (II) and (III)

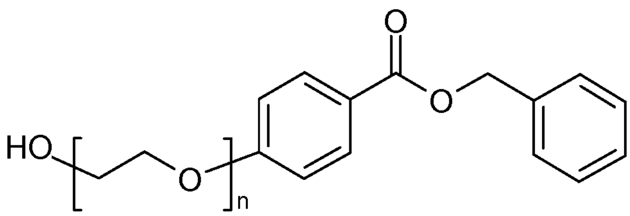

(II)

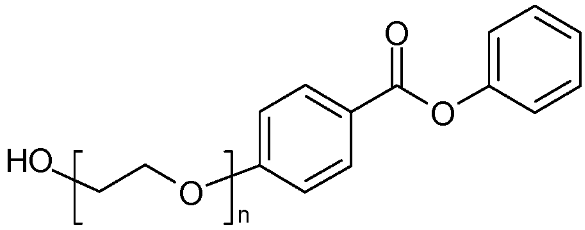

(III)

Preferably, in the inventive alkoxylates according to formula (I)

X is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy,

T is selected from the group consisting of H, $C_1$-$C_4$ alkyl, $SO_3^-$, $CH_2$-$COO^-$, sulfosuccinate and $PO_3^{2-}$, preferably is selected from the group consisting of H and $CH_3$ and more preferably is H,

| | |
|---|---|
| R1, R2 and R4 | are H, CO-NR8R3 or COO-$[X_1]_v$-R3, whereby two of the substituents R1, R2 and R4 are H and the third of these substituents is CO-NR8R3 or COO-$[X_1]_v$-R3 and preferably is COO-$[X_1]_v$-R3, |
| R8 | is H or a linear or branched alkyl group with 1 to 4 C-atoms, preferably H or $CH_3$ and more preferably H, |
| R3 | is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms or $(CH_2CH_2O)_m$phenyl, wherein m, on a molar average, is a number of from 1 to 100, preferably of from 1 to 10, more preferably of from 1 to 3 and even more preferably of from 1 to 2, and in the case, that R1 or R4 is COO-$[X_1]_v$-R3 and the other substituents of R1, R2 and R4 are H and preferably in the case that R1 is COO-$[X_1]_v$-R3 and R2 and R4 are H, R3 may also be phenyl or benzyl and preferably may also be benzyl, |
| $X_1$ | is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably $X_1$ is ethoxy, |
| u | on a molar average, is a number of from 3 to 100, preferably of from 5 to 60, more preferably of from 6 to 50, even more preferably of from 8 to 40 and particularly preferably of from 9 to 35, and |
| v | on a molar average, is a number of from 0 to 20, preferably of from 0 to 12, more preferably of from 0 to 10, even more preferably of from 0 to 8 and particularly preferably of from 0 to 7. |

In the inventive alkoxylates according to formula (I), preferably two of the substituents R1, R2 and R4 are H and the third of these substituents is COO-$[X_1]_v$-R3 and the sum u + v, on a molar average, is a number of from 3 to 100, preferably of from 5 to 60, more preferably of from 6 to 50, even more preferably of from 8 to 40 and particularly preferably of from 9 to 35.

In the inventive alkoxylates according to formula (I), more preferably two of the substituents R1, R2 and R4 are H and the third of these substituents is COOR3. In these inventive alkoxylates according to formula (I), the variable "v" in the substituent COO-$[X_1]_v$-R3 is 0.

Explicit examples for the sum u + v in the inventive alkoxylates according to formula (I), wherein two of the substituents R1, R2 and R4 are H and the third of these substituents is COO-$[X_1]_v$-R3, are, on a molar average, 9, 10, 11, 12, 13, 14, 15, 16, 17, 18, 19, 20, 21, 22, 23, 24, 25, 26, 27, 28, 29, 30, 31 or 32. Explicit examples for the variable u in the inventive alkoxylates according to formula (I), wherein two of the substituents R1, R2 and R4 are H and the third of these substituents is either CO-NR8R3 or COOR3 (i.e. COO-$[X_1]_v$-R3 wherein v is 0), are, on a molar average 9, 10, 11, 12, 13, 14, 15, 16, 17, 18, 19, 20, 21, 22, 23, 24, 25, 26, 27, 28, 29, 30, 31 or 32.

In the inventive alkoxylates according to formula (I), R4 preferably is H.

Particularly preferably, in the inventive alkoxylates according to formula (I)

| | |
|---|---|
| X | is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy, |
| T | is H, |
| R1 and R2 | are H or COOR3, whereby one of the substituents R1 and R2 is H and the other of these substituents is COOR3, |
| R3 | is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms or $(CH_2CH_2O)_m$phenyl, wherein m, on a molar average, is a number of from 1 to 100, preferably of from 1 to 10, more preferably of from 1 to 3 and even more preferably of from 1 to 2, and in the case, that R1 is COOR3 and R2 is H, R3 may also be phenyl or benzyl and preferably may also be benzyl, |
| R4 | is H, and |
| u | on a molar average, is a number of from 3 to 100, preferably of from 5 to 60, more preferably of from 6 to 50, even more preferably of from 8 to 40 and particularly preferably of from 9 to 35. |

In case T in formula (I) is selected from the group consisting of $SO_3^-$, $CH_2$-$COO^-$, sulfosuccinate and $PO_3^{2-}$ the inventive alkoxylates of formula (I) comprise a counter cation. This counter cation is preferably selected from the group consisting of alkali metal ions, alkaline earth metal ions and $NH_4^+$, more preferably from the group consisting of $Na^+$ and $NH_4^+$.

In a preferred embodiment of the invention, T in the inventive alkoxylates of the formula (I) is selected from the group consisting of $SO_3^-$, $CH_2$-$COO^-$, sulfosuccinate and $PO_3^{2-}$.

In another preferred embodiment of the invention, T in the inventive alkoxylates of the formula (I) is selected from the group consisting of H and $C_1$-$C_4$ alkyl.

More preferably, T in the inventive alkoxylates of the formula (I) is selected from the group consisting of H and $CH_3$ and even more preferably is H.

In a preferred embodiment of the invention, R1 and R4 in the inventive alkoxylates according to formula (I) are H and R2 in the inventive alkoxylates according to formula (I) is COOR3.

In another preferred embodiment of the invention, R1 in the inventive alkoxylates according to formula (I) is COOR3 and R2 and R4 in the inventive alkoxylates according to formula (I) are H.

In another preferred embodiment of the invention, R4 in the inventive alkoxylates according to formula (I) is COOR3 and R1 and R2 in the inventive alkoxylates according to formula (I) are H.

In a further preferred embodiment of the invention, R3 in the inventive alkoxylates according to formula (I) is a linear or branched saturated alkyl group with 6 to 30, preferably 8 to 22 and more preferably 12 to 18 carbon atoms or a linear or branched mono- or polyunsaturated alkenyl group with 6 to 30, preferably 8 to 22 and more preferably 12 to 18 carbon atoms and in the case that R1 is COOR3 and R2 and R4 are H, R3 may also be phenyl or benzyl and preferably may also be benzyl.

In another preferred embodiment of the invention, R3 in the inventive alkoxylates according to formula (I) is a linear or branched saturated alkyl group with 6 to 30, preferably 8 to 22 and more preferably 12 to 18 carbon atoms or a linear or branched mono- or polyunsaturated alkenyl group with 6 to 30, preferably 8 to 22 and more preferably 12 to 18 carbon atoms or $(CH_2CH_2O)_m$phenyl, wherein m, on a molar average, is a number of from 1 to 100, preferably of from 1 to 10, more preferably of from 1 to 3 and even more preferably of from 1 to 2.

In a more preferred embodiment of the invention, R3 in the inventive alkoxylates according to formula (I) is $(CH_2CH_2O)_m$phenyl, wherein m, on a molar average, is a number of from 1 to 10, preferably of from 1 to 3 and more preferably of from 1 to 2.

Even more preferably, R3 in the inventive alkoxylates according to formula (I) is a linear or branched saturated alkyl group with 6 to 30, preferably 8 to 22 and more preferably 12 to 18 carbon atoms or a linear or branched mono- or polyunsaturated alkenyl group with 6 to 30, preferably 8 to 22 and more preferably 12 to 18 carbon atoms.

Particularly preferably, R3 in the inventive alkoxylates according to formula (I) is selected from the group consisting of n-hexyl, 3-methyl-3-pentyl, n-heptyl, n-octyl, n-nonyl, n-decyl, n-undecyl, lauryl, n-tridecyl, isotridecyl, myristyl, n-pentadecyl, cetyl, palmitoleyl, n-heptadecyl, stearyl, oleyl, n-nonadecyl, arachidyl, heneicosyl, behenyl, erucyl, n-tetracosanyl, ceryl, 1-heptacosanyl, n-octacosanyl, n-nonacosanyl and myricyl.

Extraordinarily preferably, R3 in the inventive alkoxylates according to formula (I) is selected from the group consisting of stearyl, oleyl and isotridecyl. Preferred members of this group are oleyl and isotridecyl.

In particularly preferred embodiments of the invention in the inventive alkoxylates according to formula (I)

a) R1 and R4 are H, R2 is COOR3, R3 is oleyl, X is ethoxy, T is H and u, on a molar average, is a number of from 8 to 28, or
b) R1 and R4 are H, R2 is COOR3, R3 is isotridecyl, X is ethoxy, T is H and u, on a molar average, is a number of from 8 to 16, or
c) R1 and R4 are H, R2 is COOR3, R3 is isotridecyl, X is ethoxy, T is H and u, on a molar average, is a number of from 17 to 28, or
d) R1 is COOR3, R2 and R4 are H, R3 is oleyl, X is ethoxy, T is H and u, on a molar average, is a number of from 6 to 12.

Inventive alkoxylates

mentioned above under a) may be prepared according to Example 8 or 9, those
mentioned above under b) may be prepared according to Example 10, those
mentioned above under c) may be prepared according to Example 11, and those
mentioned above under d) may be prepared according to Example 7.

The isotridecyl substituent R3 preferably corresponds to the residue R3 in isotridecyl alcohol R3-OH which is produced by the hydroformulation of trimerised butene and more preferably by the hydrofomulation of trimerised n-butene.

In one preferred embodiment of the invention the inventive alkoxylates are single compounds according to formula (I).

In another preferred embodiment of the invention the inventive alkoxylates are mixtures of two or more compounds according to formula (I).

Among the single compounds according to formula (I) and the mixtures of two or more compounds according to formula (I) the mixtures are preferred.

A further subject matter of the present invention is a method of preparing inventive alkoxylates according to formula (I).

Preferably, the inventive method of preparing inventive alkoxylates of formula (I) wherein one of the substituents

R1, R2 or R4 is COO-$[X_1]_v$-R3, is performed as described in the following. In a first step ortho-, meta- or para-hydroxybenzoic acid is esterified or an ortho-, meta- or para-hydroxybenzoic acid ester of an alcohol with 1 to 4 carbon atoms and preferably the methyl esters are transesterified, in each case with an alcohol HO-$[X_1]_v$-R3, wherein $X_1$, v and R3 have the meaning given above and preferably with R3-OH at a temperature of from 140 to 250 °C and preferably of from 150 to 230 °C, in the presence of a catalyst, preferably an acid or a base, more preferably selected from the group consisting of alkali carbonate, alkaline earth carbonate, alkyl sulfonic acids and aryl sulfonic acids and even more preferably selected from the group consisting of sodium carbonate, potassium carbonate and p-toluenesulfonic acid. In a second step the ester obtained in the first step is reacted with ethylene oxide and/or propylene oxide, preferably only with ethylene oxide, though in the case that both ethylene oxide and propylene oxide are reacted, this is preferably done with more ethylene oxide than propylene oxide and in this case furthermore preferably either simultaneously or successively. The reaction is conducted in the presence of a catalyst, preferably a base or a double metal cyanide catalyst, more preferably alkali methoxide or alkali hydroxide, even more preferably sodium methoxide, potassium methoxide, sodium hydroxide or potassium hydroxide, particularly preferably sodium methoxide or potassium methoxide and extraordinarily preferably sodium methoxide, at a pressure of from 1 to 100 bar and preferably of from 2 to 10 bar, and at a temperature of from 75 to 220 °C, preferably of from 100 to 200 °C and more preferably of from 130 to 150 °C and in an optional third step the reaction product of the second step is reacted with an alkylating agent providing a $C_1$-$C_4$ alkyl group, with a carboxymethylating agent, with a sulfating agent, with a phosphating agent or with a sulfosuccinating agent.

Preferably, in the first step of the inventive method of preparing inventive alkoxylates of formula (I) wherein one of the substituents R1, R2 or R4 is COO-$[X_1]_v$-R3, ortho- or para-hydroxybenzoic acid is esterified or an ortho- or para-hydroxybenzoic acid ester of an alcohol with 1 to 4 carbon atoms and preferably the methyl esters are transesterified, in each case with an alcohol HO-$[X_1]_v$-R3, wherein $X_1$, v and R3 have the meaning given above and preferably with R3-OH.

In one preferred embodiment of the inventive method of preparing inventive alkoxylates of formula (I) wherein one of the substituents R1, R2 or R4 is COO-$[X_1]_v$-R3, the optional third step is not performed.

In another preferred embodiment of the inventive method of preparing inventive alkoxylates of formula (I) wherein one of the substituents R1, R2 or R4 is COO-$[X_1]_v$-R3, the optional third step is performed.

Preferably, the inventive method of preparing inventive alkoxylates of formula (I) wherein one of the substituents R1, R2 or R4 is CO-NR8R3, is performed as described in the following. In a first step ortho-, meta- or para-hydroxybenzoic acid chloride or ortho-, meta- or para-hydroxybenzoic acid methyl ester is reacted with an amine HNR8R3, wherein R3 and R8 have the meaning given above. In a second step the amide obtained in the first step is reacted with ethylene oxide and/or propylene oxide, preferably with only ethylene oxide, though in the case that both ethylene oxide and propylene oxide are reacted, this is preferably done with more ethylene oxide than propylene oxide and in this case furthermore preferably either simultaneously or successively. The reaction is conducted in the presence of a catalyst, preferably a base or a double metal cyanide catalyst, more preferably alkali methoxide or alkali hydroxide, even more preferably sodium methoxide, potassium methoxide, sodium hydroxide or potassium hydroxide, particularly preferably sodium methoxide or potassium methoxide and extraordinarily preferably sodium methoxide, at a pressure of from 1 to 100 bar and preferably of from 2 to 10 bar, and at a temperature of from 75 to 220 °C, preferably of from 100 to 200 °C and more preferably of from 130 to 150 °C and in an optional third step the reaction product of the second step is reacted with an alkylating agent providing a $C_1$-$C_4$ alkyl group, with a carboxymethylating agent, with a sulfating agent, with a phosphating agent or with a sulfosuccinating agent. Preferably, in the first step of the inventive method of preparing inventive alkoxylates of formula (I) wherein one of the substituents R1, R2 or R4 is CO-NR8R3, ortho- or para-hydroxybenzoic acid chloride or ortho- or para-hydroxybenzoic acid methyl ester is reacted with an amine HNR8R3, wherein R3 and R8 have the meaning given above.

In one preferred embodiment of the inventive method of preparing inventive alkoxylates of formula (I) wherein one of the substituents R1, R2 or R4 is CO-NR8R3, the optional third step is not performed.

In another preferred embodiment of the inventive method of preparing inventive alkoxylates of formula (I) wherein one of the substituents R1, R2 or R4 is CO-NR8R3, the optional third step is performed.

In the optional third step of the inventive method of preparing inventive alkoxylates of formula (I) the reaction product of the second step is reacted with an alkylating agent providing a $C_1$-$C_4$ alkyl group, with a carboxymethylating agent, with a sulfating agent, with a phosphating agent or with a sulfosuccinating agent.

Suitable alkylating agents providing $C_1$-$C_4$ alkyl groups are e.g. dialkylsulfates with $C_1$-$C_4$ alkyl groups and preferably dimethylsulfate, $C_1$-$C_4$ alkylhalogenides, preferably $C_1$-$C_4$ alkylchlorides, -bromides or -iodides and more preferably $C_1$-$C_4$ alkylchlorides, $C_1$-$C_4$ alkyltosylates or $C_1$-$C_4$ alkylmesylates.

Suitable carboxymethylating agents are e.g. chloroacetic acid, bromoacetic acid, iodoacetic acid or their salts and preferably chloroacetic acid.

Suitable sulfating agents are e.g. $SO_3$ or amidosulfonic acid.

Suitable phosphating agents are e.g. polyphosphoric acid, phosphorous oxides such as $P_2O_5$, $PCl_3$ in combination with an oxidation reaction and $PCl_5$ or $POCl_3$ in combination with a hydrolysis.

Suitable sulfosuccinating agents are e.g. maleic anhydride in combination with sulfite.

Reactions of a compound comprising an OH group with an alkylating agent providing a $C_1$-$C_4$ alkyl group, with a carboxymethylating agent, with a sulfating agent, with a phosphating agent or with a sulfosuccinating agent are already known. Reactions of this kind are e.g. described in WO 2008/138486 A1.

Further preferred embodiments of the invention result from the combination of two or more of the preferred, more preferred, even more preferred, particularly preferred and extraordinarily preferred embodiments of the invention.

The examples below are intended to illustrate the invention in detail without, however, limiting it thereto.

Examples

Benzyl salicylate was used as purchased from Sigma Aldrich.

Oleyl alcohol (HD-Ocenol® 70/75 V) was used as purchased from BASF.

Isotridecyl alcohol (Marlipal® 013) was used as purchased from Sasol.

Isotridecyl alcohol and oleyl alcohol were used in technical grade quality and their molecular masses were determined prior to use by measuring the hydroxyl value (OH-value) and subsequently calculating the molecular weight (per hydroxyl function, "Gebrauchsmol"). In this case the OH-value may be measured according to DIN 53240.

The degree of alkoxylation of the inventive alkoxylates may be checked using NMR spectroscopy. The degree of ethoxylation of described examples was checked using $^1$H-NMR spectroscopy in analogy to the method described in R. Stevanova, D. Rankoff, S. Panayotova, S.L. Spassov, J. Am. Oil Chem. Soc., 65, 1516-1518 (1988). For this purpose, the samples are derivatised by reacting them with trichloro acetyl isocyanate and measured as solutions in deuterated chloroform containing 1 weight-% (1 wt.-%) of tetramethyl silane as internal standard.

Residual contents of salicylic acid and 4-hydroxybenzoic acid were determined by High Performance Liquid Chromatography (HPLC) after calibration with the pure materials. The analyses were conducted using an Ascentis Express RP-Amide column (150 mm length and 4.6 mm diameter with 2.7 $\mu$m silica particle size). The compounds were detected using a diode array detector (DAD, $\lambda$1 = 254 nm (hydroxybenzoic acid), $\lambda$2 = 303 nm (hydroxybenzoic acid). An eluent mixture of A: 0.1 % (v/v) formic acid in water and B: acetonitrile/methanol 50/50 (v/v) was used in the following profile: 0-3 minutes: 30 % (v/v) B, 3-20 minutes: 80 % (v/v) B, 20-22 min: 100 % (v/v) B, 22-30 minutes: 30 % (v/v) B. The system was operated at a temperature of 40 °C with a flow rate of 1.4 ml/minute. For the sample preparation, 50 mg of the sample were dissolved in 10 ml of eluent B.

The esterification reactions of examples 1-4 were controlled by determining the residual content of oleyl alcohol and isotridecyl alcohol by GC-FID. Calibration was performed with pure starting materials. Gas chromatography (GC) was performed using a Hewlett Packard GC 6890 with autosampler, coupled with a flame-ionisation detector (fid). Samples were separated on a 25 m x 0.32 mm, 0.52 $\mu$m film DB-5 column. The column temperature was initially held at 40 °C for 2 minutes, then the temperature was raised to 250 °C at a rate of 10 °C per minute and held for 6.5 minutes. The injector temperature was maintained at 250 °C, the detector temperature was maintained at 250 °C and the injection volume was 1.0 $\mu$L in the split mode. Helium was used as a carrier gas with a constant pressure of 0.9 bar. The samples were prepared by diluting 500 mg of sample with 5 ml of methanol.

Thin layer chromatography (TLC) was performed using TLC Silica Gel 60 F254 plates from Merck. The aromatic compounds were detected by UV light (254 and 366 nm simultaneously). As the eluent, a mixture of hexanes/ethyl acetate = 1:1 was used.

Example 1

379.4 g (1.4 mol) of oleyl alcohol, 213.0 g (1.4 mol) of methyl salicylate and 6.0 g (43.4 mmol) of potassium carbonate were dissolved in 200 ml of xylene in a 1 liter round bottom flask equipped with a KPG stirrer, a reflux condenser and a thermometer. The reaction mixture was heated to reflux (150 - 166 °C) under nitrogen atmosphere for 11 hours. After this time, the status of the reaction was checked by thin layer chromatography (TLC; ethyl acetate : heptane = 3:2) and no methyl salicylate was found.

The reaction mixture was cooled to room temperature, washed three times with 200 ml of saturated aqueous sodium bicarbonate solution and three times with 200 ml of water. The organic phase was dried with $MgSO_4$ and the solvent was removed under reduced pressure. After the described workup, 409.6 g of the product was obtained as a dark brown oil. The residual content of methyl salicylate was < 0.1 wt.-% as determined by gas chromatography (GC).

Example 2

352.3 g (1.3 mol) of oleyl alcohol, 179.6 g (1.3 mol) of 4-hydroxybenzoic acid and 1.2 g (6.5 mmol) of p-toluenesulfonic acid were added into a 1 liter round bottom flask equipped with a KPG stirrer, a distillation bridge, a reflux condenser and a thermometer. The reaction mixture was heated to 170 °C under nitrogen atmosphere. The reaction was stirred at 170 °C for 8 hours and during that time the reaction water was distilled off. Afterwards, the reaction

mixture was heated to 200 °C. The reaction mixture was stirred at 200 °C for 8 hours and during that time the reaction water was distilled off.

The reaction mixture was cooled to room temperature and 483.1 g of the product were obtained and analysed. The residual content of 4-hydroxybenzoic acid was 0.25 wt.-% as determined by high performance liquid chromatography (HPLC) and the residual content of oleyl alcohol was 0.1 wt.-% as determined by gas chromatography (GC).

Example 3

303.8 g (1.5 mol) of isotridecyl alcohol, 207.2 g (1.5 mol) of 4-hydroxybenzoic acid and 1.4 g (7.5 mmol) of p-toluenesulfonic acid were added into a 1 liter round bottom flask equipped with a KPG stirrer, a distillation bridge, a reflux condenser and a thermometer. The reaction mixture was heated to 170 °C under nitrogen atmosphere. The reaction was stirred at 170 °C for 8 hours and during that time the reaction water was distilled off. Afterwards, the reaction mixture was heated to 200 °C. The reaction mixture was stirred at 200 °C for 8 hours and during that time the reaction water was distilled off.

The reaction mixture was cooled to room temperature and 444.1 g of the product were obtained and analysed. The residual content of 4-hydroxybenzoic acid was 0.64 wt.-% as determined by HPLC and the residual content of isotridecyl alcohol was < 0.1 wt.-% as determined by gas chromatography (GC).

General procedure for the ethoxylation of hydroxyaromatic esters:

The hydroxyaromatic ester was filled into a dry and clean lab autoclave. Sodium methoxide solution in methanol was added under stirring and then the autoclave was purged with nitrogen. After a successful pressure test, the pressure in the autoclave was again reduced to atmospheric pressure. Then full vacuum was applied and the reaction mixture was heated up to 120 °C for removal of methanol. This drying was continued for 2 hours at 120 °C. After that, the vacuum was compensated with nitrogen. The reaction mixture was heated to 140 °C. At this temperature a safe amount of ethylene oxide (EO) was added and the pressure observed until the reaction started (pressure decreased). In the following 7 to 17 hours the remaining ethylene oxide was added at 140 °C and stirring was continued for one to two hours to complete the reaction. Then the reaction mixture was cooled down to 100 °C and vacuum was applied for 30 minutes to remove residual ethylene oxide. After that, the vacuum was compensated with nitrogen, the reaction mixture cooled down to 80 °C and filled into a flask.

Example 4 - ethoxylation of benzyl salicylate with 10 equivalents of EO

156.8 g of benzyl salicylate and 1.2 g of sodium methoxide solution (30 wt.-% in methanol) were reacted with 302.3 g of ethylene oxide (10 mol EO/mol) as described in the general procedure for the ethoxylation of hydroxyaromatic esters. 206.7 g of the product (brown oil) were discharged out of the reactor.

Example 5 - ethoxylation of benzyl salicylate with 20 equivalents of EO

The remaining product of example 4 (252.4 g, calculated) was reacted with additional 166.2 g of ethylene oxide (10 mol EO/mol) as described in the general procedure for the ethoxylation of hydroxyaromatic esters. 206.9 g of the product (brown oil) were discharged out of the reactor.

Example 6 - ethoxylation of benzyl salicylate with 30 equivalents of EO

The remaining product of example 5 (211.7 g, calculated) was reacted with additional 84.1 g of ethylene oxide (10 mol EO/mol) as described in the general procedure for the ethoxylation of hydroxyaromatic esters. 295.8 g of the product (brown oil) were obtained.

Example 7 - ethoxylation of the hydroxyaromatic ester of example 1 with 10 equivalents of EO (DMC (double metal cyanide) catalysis)

165.0 g of the hydroxyaromatic ester of example 1 was filled into a dry and clean lab autoclave. 0.08 g of Arcol Catalyst 3 (Bayer) and two droplets of phosphoric acid (40 wt.-% in water) were added under stirring, and then the autoclave was purged with nitrogen. After a successful pressure test, the pressure in the autoclave was again reduced to atmospheric pressure. Then full vacuum was applied and the reaction mixture was heated up to 140 °C. When the temperature was reached, the vacuum was compensated with nitrogen. A safe amount (13 g) of ethylene oxide was added. Additional 0.125 g Arcol Catalyst 3 was added, because no evidence that the reaction had begun (i.e. no temperature increase) was observed. After addition of further 26 g ethylene oxide the typical conversion started and the rest of the 199.0 g ethylene oxide was added. The reaction mixture was stirred for one hour after completed addition. Then the reaction mixture was cooled down to 100 °C and vacuum was applied for 30 minutes to remove residual ethylene oxide. After that, the vacuum was compensated with nitrogen and the reaction mixture cooled down to 80 °C. 173.0 g of the product (brown oil) were discharged out of the reactor.

Example 8 - ethoxylation of the hydroxyaromatic ester of example 2 with 10 equivalents of EO

101.5 g of the hydroxyaromatic ester of example 2 and 1.4 g of sodium methoxide solution (30 wt.-% in methanol) were reacted with 114.9 g of ethylene oxide (10 mol EO/mol) as described in the general procedure for the ethoxylation of hydroxyaromatic esters. 97.1 g of the product (brown oil) were discharged out of the reactor.

Example 9 - ethoxylation of the hydroxyaromatic ester of example 2 with 20 equivalents of EO

The remaining product of example 8 (119.3 g, calculated) was reacted with additional 63.4 g of ethylene oxide (10 mol EO/mol) as described in the general procedure for the ethoxylation of hydroxyaromatic esters. 182.7 g of the product (brown oil) were obtained.

Example 10 - ethoxylation of the hydroxyaromatic ester of example 3 with 10 equivalents of EO

65.4 g of the hydroxyaromatic ester of example 3 and 1.2 g of sodium methoxide solution (30 wt.-% in methanol) were reacted with 90.4 g of ethylene oxide (10 mol EO/mol) as described in the general procedure for the ethoxylation of hydroxyaromatic esters. 64.2 g of the product (brown oil) were discharged out of the reactor.

Example 11 - ethoxylation of the hydroxyaromatic ester of example 3 with 20 equivalents of EO

The remaining product of example 10 (91.6 g, calculated) was reacted with additional 53.2 g of ethylene oxide (10 mol EO/mol) as described in the general procedure for the ethoxylation of hydroxyaromatic esters. 144.8 g of the product (brown oil) were obtained.

An aqueous liquid laundry detergent of the following formulation was prepared:

Table 1: Liquid laundry detergent formulation

| Ingredient | weight-% |
|---|---|
| Mono propylene glycol | 2.2 |
| Triethylamine | 1.5 |
| $C_{12}$-$C_{15}$ alcohol ethoxylate with 7 moles of ethylene oxide | 1.2 |
| Linear alkyl benzene sulfonate | 4.6 |
| Sodium laureth ether sulphate with 1 moles of ethylene oxide | 5.8 |
| Citric acid | 2.0 |
| $CaCl_2$ dihydrate | 0.2 |
| NaCl | 0.2 |
| Tinopal® CBS-X (fluorescer BASF) | 0.3 |
| Sodium Hydroxide | to pH = 8.4 |
| Exemplary dispersants | see text |
| Water | balance |

Application Example 1 - Anti-Redeposition Benefit

The formulation was used to wash eight 5x5 cm knitted cotton cloth pieces in a tergotometer set at 200 rpm (revolutions per minute). A one hour wash was conducted in 800 ml of 26° French Hard water at 20 °C, with 2.3 g/l of the formulation. To simulate particulate soil that could redeposit 0.04 g/l of 100 % compressed carbon black (ex Alfa

Aesar) was added to the wash liquor. To simulate oily sebaceous soil 7.2 g of an SBL2004 soil strip (ex Warwick Equest) was added to the wash liquor.

Once the wash had been completed the cotton swatches were rinsed once in 400 ml clean water, removed, dried and the colour measured on a reflectometer and expressed as the CIE L*a*b* values. The anti-redeposition benefit was expressed as the ΔL value:

$$\Delta L = L(\text{dispersant}) - L(\text{control})$$

The larger the ΔL value the greater the prevention of deposition of the carbon black soil. 95 % confidence limits based on the 8 separate cotton swatches were calculated. Formulations were made with and without the addition of 8.7 wt.-% of the dispersant of Table 2. The results are given in Table 2.

Table 2: Anti-redeposition benefit

| Exemplary dispersant | ΔL | 95 % |
|---|---|---|
| Example 8 | 9.5 | 0.4 |
| Example 9 | 10.7 | 0.3 |
| Example 10 | 11.7 | 0.3 |
| Example 11 | 4.2 | 0.2 |
| Example 7 | 4.1 | 0.2 |

The dispersants enhance anti-redeposition.

## Claims

1. Alkoxylate according to the following formula (I)

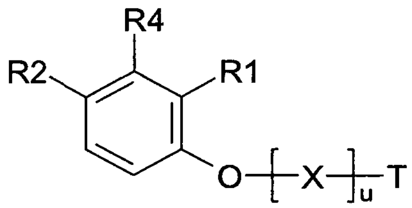

(I)

wherein

X is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy,

T is selected from the group consisting of H, $C_1$-$C_4$ alkyl, $SO_3^-$, $CH_2$-$COO^-$, sulfosuccinate and $PO_3^{2-}$, preferably is selected from the group consisting of H and $CH_3$ and more preferably is H,

R1, R2 and R4 are H, CO-NR8R3 or COO-$[X_1]_v$-R3, whereby two of the substituents R1, R2 and R4 are H and the third of these substituents is CO-NR8R3 or COO-$[X_1]_v$-R3 and preferably is COO-$[X_1]_v$-R3,

R8 is H or a linear or branched alkyl group with 1 to 4 C-atoms, preferably H or $CH_3$ and more preferably H,

R3 is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms, an aryl group with 6 to 10 carbon atoms, preferably phenyl, a group $(CH_2CH_2O)_m$aryl, wherein the aryl group comprises 6 to 10 carbon atoms and m, on a molar average, is a number of from 1 to 100, preferably of from 1 to 10, more preferably of from 1 to 3 and even more preferably of from 1 to 2, and preferably $(CH_2CH_2O)_m$aryl is $(CH_2CH_2O)_m$phenyl, or an aryl-substituted linear or branched $C_1$-$C_3$ alkyl group wherein the aryl group comprises 6 to 10 carbon atoms, preferably benzyl, but in the case, that R1 and R4 are H and R2 is COOR3, R3 can only be an alkyl group, an alkenyl group or a group

$(CH_2CH_2O)_m$aryl as defined above and in this case R3 preferably is an alkyl group or an alkenyl group as defined above,
$X_1$ is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably $X_1$ is ethoxy,
u on a molar average, is a number of from 3 to 100, preferably of from 5 to 60, more preferably of from 6 to 50, even more preferably of from 8 to 40 and particularly preferably of from 9 to 35, and
v on a molar average, is a number of from 0 to 20, preferably of from 0 to 12, more preferably of from 0 to 10, even more preferably of from 0 to 8 and particularly preferably of from 0 to 7.

**2.** Alkoxylate according to claim 1, **characterised in that**

X is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy,
T is selected from the group consisting of H, $C_1$-$C_4$ alkyl, $SO_3^-$, $CH_2$-$COO^-$, sulfosuccinate and $PO_3^{2-}$, preferably is selected from the group consisting of H and $CH_3$ and more preferably is H,
R1, R2 and R4 are H, CO-NR8R3 or COO-$[X_1]_v$-R3, whereby two of the substituents R1, R2 and R4 are H and the third of these substituents is CO-NR8R3 or COO-$[X_1]_v$-R3 and preferably is COO-$[X_1]_v$-R3,
R8 is H or a linear or branched alkyl group with 1 to 4 C-atoms, preferably H or $CH_3$ and more preferably H,
R3 is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms or $(CH_2CH_2O)_m$phenyl, wherein m, on a molar average, is a number of from 1 to 100, preferably of from 1 to 10, more preferably of from 1 to 3 and even more preferably of from 1 to 2, and in the case, that R1 or R4 is COO-$[X_1]_v$-R3 and the other substituents of R1, R2 and R4 are H and preferably in the case that R1 is COO-$[X_1]_v$-R3 and R2 and R4 are H, R3 may also be phenyl or benzyl and preferably may also be benzyl,
$X_1$ is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably $X_1$ is ethoxy,
u on a molar average, is a number of from 3 to 100, preferably of from 5 to 60, more preferably of from 6 to 50, even more preferably of from 8 to 40 and particularly preferably of from 9 to 35, and
v on a molar average, is a number of from 0 to 20, preferably of from 0 to 12, more preferably of from 0 to 10, even more preferably of from 0 to 8 and particularly preferably of from 0 to 7.

**3.** Alkoxylate according to claim 1 or 2, **characterised in that** two of the substituents R1, R2 and R4 are H and the third of these substituents is COO-$[X_1]_v$-R3 and the sum u + v, on a molar average, is a number of from 3 to 100, preferably of from 5 to 60, more preferably of from 6 to 50, even more preferably of from 8 to 40 and particularly preferably of from 9 to 35.

**4.** Alkoxylate according to one or more of claims 1 to 3, **characterised in that** two of the substituents R1, R2 and R4 are H and the third of these substituents is COOR3.

**5.** Alkoxylate according to one or more of claims 1 to 4, **characterised in that** R4 is H.

**6.** Alkoxlate according to one or more of claims 1 to 5, **characterised in that**

X is selected from ethoxy and mixtures of ethoxy and propoxy groups, preferably is selected from ethoxy and mixtures of ethoxy and propoxy groups where the number of ethoxy groups in the mixtures is greater than the number of propoxy groups and more preferably X is ethoxy,
T is H,
R1 and R2 are H or COOR3, whereby one of the substituents R1 and R2 is H and the other of these substituents is COOR3,
R3 is a linear or branched saturated alkyl group with 6 to 30, preferably 8 to 22 and more preferably 12 to 18 carbon atoms, a linear or branched mono- or polyunsaturated alkenyl group with 6 to 30, preferably 8 to 22 and more preferably 12 to 18 carbon atoms or $(CH_2CH_2O)_m$phenyl, wherein m, on a molar average, is a number of from 1 to 100, preferably of from 1 to 10, more preferably of from 1 to 3 and even more preferably of from 1 to 2, and in the case, that R1 is COOR3 and R2 is H, R3 may also be phenyl or benzyl and preferably may also

be benzyl,
R4 is H, and
u on a molar average, is a number of from 3 to 100, preferably of from 5 to 60, more preferably of from 6 to 50, even more preferably of from 8 to 40 and particularly preferably of from 9 to 35.

**7.** Alkoxylate according to one or more of claims 1 to 6, **characterised in that** R1 and R4 are H and R2 is COOR3.

**8.** Alkoxylate according to one or more of claims 1 to 6, **characterised in that** R1 is COOR3 and R2 and R4 are H.

**9.** Alkoxylate according to one or more of claims 1 to 8, **characterised in that** R3 is a linear or branched saturated alkyl group with 6 to 30, preferably 8 to 22 and more preferably 12 to 18 carbon atoms or a linear or branched mono- or polyunsaturated alkenyl group with 6 to 30, preferably 8 to 22 and more preferably 12 to 18 carbon atoms and in the case that R1 is COOR3 and R2 and R4 are H, R3 may also be phenyl or benzyl and preferably may also be benzyl.

**10.** Alkoxylate according to one or more of claims 1 to 8, **characterised in that** R3 is a linear or branched saturated alkyl group with 6 to 30, preferably 8 to 22 and more preferably 12 to 18 carbon atoms or a linear or branched mono- or polyunsaturated alkenyl group with 6 to 30, preferably 8 to 22 and more preferably 12 to 18 carbon atoms or $(CH_2CH_2O)_m$phenyl, wherein m, on a molar average, is a number of from 1 to 100, preferably of from 1 to 10, more preferably of from 1 to 3 and even more preferably of from 1 to 2.

**11.** Alkoxylate according to one or more of claims 1 to 8 and 10, **characterised in that** R3 is $(CH_2CH_2O)_m$phenyl, wherein m, on a molar average, is a number of from 1 to 10, preferably of from 1 to 3 and more preferably of from 1 to 2.

**12.** Alkoxylate according to one or more of claims 1 to 10, **characterised in that** R3 is a linear or branched saturated alkyl group with 6 to 30, preferably 8 to 22 and more preferably 12 to 18 carbon atoms or a linear or branched mono- or polyunsaturated alkenyl group with 6 to 30, preferably 8 to 22 and more preferably 12 to 18 carbon atoms.

**13.** Alkoxylate according to one or more of claims 1 to 10 and 12, **characterised in that** R3 is selected from the group consisting of n-hexyl, 3-methyl-3-pentyl, n-heptyl, n-octyl, n-nonyl, n-decyl, n-undecyl, lauryl, n-tridecyl, isotridecyl, myristyl, n-pentadecyl, cetyl, palmitoleyl, n-heptadecyl, stearyl, oleyl, n-nonadecyl, arachidyl, heneicosyl, behenyl, erucyl, n-tetracosanyl, ceryl, 1-heptacosanyl, n-octacosanyl, n-nonacosanyl and myricyl.

**14.** Alkoxylate according to one or more of claims 1 to 10, 12 and 13, **characterised in that** R3 is selected from the group consisting of stearyl, oleyl and isotridecyl and preferably is selected from the group consisting of oleyl and isotridecyl.

**15.** Alkoxylate according to one or more of claims 1 to 10 and 12 to 14, **characterised in that**

a) R1 and R4 are H, R2 is COOR3, R3 is oleyl, X is ethoxy, T is H and u, on a molar average, is a number of from 8 to 28, or
b) R1 and R4 are H, R2 is COOR3, R3 is isotridecyl, X is ethoxy, T is H and u, on a molar average, is a number of from 8 to 16, or
c) R1 and R4 are H, R2 is COOR3, R3 is isotridecyl, X is ethoxy, T is H and u, on a molar average, is a number of from 17 to 28, or
d) R1 is COOR3, R2 and R4 are H, R3 is oleyl, X is ethoxy, T is H and u, on a molar average, is a number of from 6 to 12.

**16.** Alkoxylate according to one or more of claims 1 to 15, **characterised in that** it is a mixture of two or more compounds according to formula (I).

**17.** Method of preparing an alkoxylate according to one or more of claims 1 to 16, **characterised in that** one of the substituents R1, R2 or R4 in formula (I) is COO-$[X_1]_v$-R3 and that in a first step ortho-, meta- or para-hydroxybenzoic acid is esterified or an ortho-, meta- or para-hydroxybenzoic acid ester of an alcohol with 1 to 4 carbon atoms is transesterified, in each case with an alcohol HO-$[X_1]_v$-R3, wherein $X_1$, v and R3 have the meaning given in one or more of claims 1 to 16 at a temperature of from 140 to 250 °C in the presence of a catalyst, and in a second step the ester obtained in the first step is reacted with ethylene oxide and/or propylene oxide in the presence of a catalyst at a pressure of from 1 to 100 bar and at a temperature of from 75 to 220 °C and in an optional third step the reaction

product of the second step is reacted with an alkylating agent providing a $C_1$-$C_4$ alkyl group, with a carboxymethylating agent, with a sulfating agent, with a phosphating agent or with a sulfosuccinating agent.

**18.** Method of preparing an alkoxylate according to one or more of claims 1, 2, 5, 9 to 14 and 16, **characterised in that** one of the substituents R1, R2 or R4 in formula (I) is CO-NR8R3 and that in a first step ortho-, meta- or para-hydroxybenzoic acid chloride or ortho-, meta- or para-hydroxybenzoic acid methyl ester is reacted with an amine HNR8R3, wherein R3 and R8 have the meaning given in one or more of claims 1, 2, 5, 9 to 14 and 16 and in a second step the amide obtained in the first step is reacted with ethylene oxide and/or propylene oxide in the presence of a catalyst at a pressure of from 1 to 100 bar and at a temperature of from 75 to 220 °C and in an optional third step the reaction product of the second step is reacted with an alkylating agent providing a $C_1$-$C_4$ alkyl group, with a carboxymethylating agent, with a sulfating agent, with a phosphating agent or with a sulfosuccinating agent.

## Patentansprüche

**1.** Alkoxylat gemäß der folgenden Formel (I)

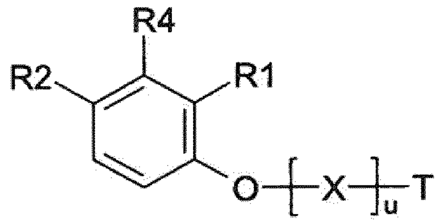

(I)

wobei

X aus Ethoxy und Mischungen von Ethoxy- und Propoxygruppen ausgewählt ist und vorzugsweise aus Ethoxy und Mischungen von Ethoxy- und Propoxygruppen, wobei die Zahl der Ethoxygruppen in den Mischungen größer ist als die Zahl der Propoxygruppen ausgewählt ist und weiter bevorzugt X für Ethoxy steht,
T aus der Gruppe bestehend aus H, $C_1$-$C_4$-Alkyl, $SO_3^-$, $CH_2$-$COO^-$, Sulfosuccinat und $PO_3^{2-}$ ausgewählt ist, vorzugsweise aus der Gruppe bestehend aus H und $CH_3$ ausgewählt ist und weiter bevorzugt für H steht,
R1, R2 und R4 für H, CO-NR8R3 oder COO-$[X_1]_v$-R3 stehen, wobei zwei der Substituenten R1, R2 und R4 für H stehen und der Dritte dieser Substituenten für CO-NR8R3 oder COO-$[X_1]_v$-R3 und vorzugsweise für COO-$[X_1]_v$-R3 steht,
R8 für H oder eine lineare oder verzweigte Alkylgruppe mit 1 bis 4 C-Atomen, vorzugsweise H oder $CH_3$ und weiter bevorzugt H steht,
R3 für eine lineare oder verzweigte gesättigte Alkylgruppe mit 6 bis 30, vorzugsweise 8 bis 22 und weiter bevorzugt 12 bis 18 Kohlenstoffatomen, eine lineare oder verzweigte ein- oder mehrfach ungesättigte Alkenylgruppe mit 6 bis 30, vorzugsweise 8 bis 22 und weiter bevorzugt 12 bis 18 Kohlenstoffatomen, eine Arylgruppe mit 6 bis 10 Kohlenstoffatomen, vorzugsweise Phenyl, eine Gruppe $(CH_2CH_2O)_m$Aryl, wobei die Arylgruppe 6 bis 10 Kohlenstoffatome umfasst und m im molaren Durchschnitt für eine Zahl von 1 bis 100, vorzugsweise von 1 bis 10, weiter bevorzugt von 1 bis 3 und noch weiter bevorzugt von 1 bis 2 steht, und vorzugsweise $(CH_2CH_2O)_m$Aryl $(CH_2CH_2O)_m$Phenyl ist, oder eine arylsubstituierte lineare oder verzweigte $C_1$-$C_3$-Alkylgruppe, wobei die Aryl Gruppe 6 bis 10 Kohlenstoffatome umfasst, vorzugsweise Benzyl, steht, aber in dem Fall, dass R1 und R4 für H stehen und R2 für COOR3 steht, R3 nur für eine Alkylgruppe, eine Alkenylgruppe oder eine Gruppe $(CH_2CH_2O)_m$Aryl gemäß obiger Definition stehen kann und in diesem Fall R3 vorzugsweise für eine Alkylgruppe oder eine Alkenylgruppe gemäß obiger Definition steht,
$X_1$ aus Ethoxy und Mischungen von Ethoxy- und Propoxygruppen ausgewählt ist und vorzugsweise aus Ethoxy und Mischungen von Ethoxy- und Propoxygruppen, wobei die Zahl der Ethoxygruppen in den Mischungen größer ist als die Zahl der Propoxygruppen, ausgewählt ist und weiter bevorzugt $X_1$ für Ethoxy steht,
u im molaren Durchschnitt für eine Zahl von 3 bis 100, vorzugsweise von 5 bis 60, weiter bevorzugt von 6 bis 50, noch weiter bevorzugt von 8 bis 40 und besonders bevorzugt von 9 bis 35 steht und
v im molaren Durchschnitt für eine Zahl von 0 bis 20, vorzugsweise von 0 bis 12, weiter bevorzugt von 0 bis 10, noch weiter bevorzugt von 0 bis 8 und besonders bevorzugt von 0 bis 7 steht.

**2.** Alkoxylat nach Anspruch 1, **dadurch gekennzeichnet, dass**

X aus Ethoxy und Mischungen von Ethoxy- und Propoxygruppen ausgewählt ist und vorzugsweise aus Ethoxy und Mischungen von Ethoxyund Propoxygruppen, wobei die Zahl der Ethoxygruppen in den Mischungen größer ist als die Zahl der Propoxygruppen, ausgewählt ist und weiter bevorzugt X für Ethoxy steht,
T aus der Gruppe bestehend aus H, $C_1$-$C_4$-Alkyl, $SO_3^-$, $CH_2$-$COO^-$, Sulfosuccinat und $PO_3^{2-}$ ausgewählt ist, vorzugsweise aus der Gruppe bestehend aus H und $CH_3$ ausgewählt ist und weiter bevorzugt für H steht,
R1, R2 und R4 für H, CO-NR8R3 oder COO-$[X_1]_v$-R3 stehen, wobei zwei der Substituenten R1, R2 und R4 für H stehen und der Dritte dieser Substituenten für CO-NR8R3 oder COO-$[X_1]_v$-R3 und vorzugsweise für COO-$[X_1]_v$-R3 steht,
R8 für H oder eine lineare oder verzweigte Alkylgruppe mit 1 bis 4 C-Atomen, vorzugsweise H oder $CH_3$ und weiter bevorzugt H steht,
R3 für eine lineare oder verzweigte gesättigte Alkylgruppe mit 6 bis 30, vorzugsweise 8 bis 22 und weiter bevorzugt 12 bis 18 Kohlenstoffatomen, eine lineare oder verzweigte ein- oder mehrfach ungesättigte Alkenylgruppe mit 6 bis 30, vorzugsweise 8 bis 22 und weiter bevorzugt 12 bis 18 Kohlenstoffatomen oder $(CH_2CH_2O)_m$Phenyl, wobei m im molaren Durchschnitt für eine Zahl von 1 bis 100, vorzugsweise von 1 bis 10, weiter bevorzugt von 1 bis 3 und noch weiter bevorzugt von 1 bis 2 steht, steht und in dem Fall, dass R1 oder R4 für COO-$[X_1]_v$-R3 steht und die anderen Substituenten von R1, R2 und R4 für H stehen, und vorzugsweise in dem Fall, dass R1 für COO-$[X_1]_v$-R3 steht und R2 und R4 für H stehen, R3 auch für Phenyl oder Benzyl und vorzugsweise auch für Benzyl stehen kann,
$X_1$ aus Ethoxy und Mischungen von Ethoxy- und Propoxygruppen ausgewählt ist und vorzugsweise aus Ethoxy und Mischungen von Ethoxy-und Propoxygruppen, wobei die Zahl der Ethoxygruppen in den Mischungen größer ist als die Zahl der Propoxy-gruppen, ausgewählt ist und weiter bevorzugt $X_1$ für Ethoxy steht,
u im molaren Durchschnitt für eine Zahl von 3 bis 100, vorzugsweise von 5 bis 60, weiter bevorzugt von 6 bis 50, noch weiter bevorzugt von 8 bis 40 und besonders bevorzugt von 9 bis 35 steht und
v im molaren Durchschnitt für eine Zahl von 0 bis 20, vorzugsweise von 0 bis 12, weiter bevorzugt von 0 bis 10, noch weiter bevorzugt von 0 bis 8 und besonders bevorzugt von 0 bis 7 steht.

**3.** Alkoxylat nach Anspruch 1 oder 2, **dadurch gekennzeichnet, dass** zwei der Substituenten R1, R2 und R4 für H stehen und der Dritte dieser Substituenten für COO-$[X_1]_v$-R3 steht und die Summe u + v im molaren Durchschnitt für eine Zahl von 3 bis 100, vorzugsweise von 5 bis 60, weiter bevorzugt von 6 bis 50, noch weiter bevorzugt von 8 bis 40 und besonders bevorzugt von 9 bis 35 steht.

**4.** Alkoxylat nach einem oder mehreren der Ansprüche 1 bis 3, **dadurch gekennzeichnet, dass** zwei der Substituenten R1, R2 und R4 für H stehen und der Dritte dieser Substituenten für COOR3 steht.

**5.** Alkoxylat nach einem oder mehreren der Ansprüche 1 bis 4, **dadurch gekennzeichnet, dass** R4 für H steht.

**6.** Alkoxylat nach einem oder mehreren der Ansprüche 1 bis 5, **dadurch gekennzeichnet, dass**

X aus Ethoxy und Mischungen von Ethoxy- und Propoxygruppen ausgewählt ist und vorzugsweise aus Ethoxy und Mischungen von Ethoxy- und Propoxygruppen, wobei die Zahl der Ethoxygruppen in den Mischungen größer ist als die Zahl der Propoxygruppen, ausgewählt ist und weiter bevorzugt X für Ethoxy steht,
T für H steht,
R1 und R2 für H oder COOR3 stehen, wobei einer der Substituenten R1 und R2 für H steht und der andere dieser Substituenten für COOR3 steht,
R3 für eine lineare oder verzweigte gesättigte Alkylgruppe mit 6 bis 30, vorzugsweise 8 bis 22 und weiter bevorzugt 12 bis 18 Kohlenstoffatomen, eine lineare oder verzweigte ein- oder mehrfach ungesättigte Alkenylgruppe mit 6 bis 30, vorzugsweise 8 bis 22 und weiter bevorzugt 12 bis 18 Kohlenstoffatomen oder $(CH_2CH_2O)_m$Phenyl, wobei m im molaren Durchschnitt für eine Zahl von 1 bis 100, vorzugsweise von 1 bis 10, weiter bevorzugt von 1 bis 3 und noch weiter bevorzugt von 1 bis 2 steht, steht und in dem Fall, dass R1 für COOR3 steht und R2 für H steht, R3 auch für Phenyl oder Benzyl und vorzugsweise auch für Benzyl stehen kann,
R4 für H steht und
u im molaren Durchschnitt für eine Zahl von 3 bis 100, vorzugsweise von 5 bis 60, weiter bevorzugt von 6 bis 50, noch weiter bevorzugt von 8 bis 40 und besonders bevorzugt von 9 bis 35 steht.

**7.** Alkoxylat hat nach einem oder mehreren der Ansprüche 1 bis 6, **dadurch gekennzeichnet, dass** R1 und R4 für H stehen und R2 für COOR3 steht.

**8.** Alkoxylat nach einem oder mehreren der Ansprüche 1 bis 6, **dadurch gekennzeichnet, dass** R1 für COOR3 steht

und R2 und R4 für H stehen.

**9.** Alkoxylat nach einem oder mehreren der Ansprüche 1 bis 8, **dadurch gekennzeichnet, dass** R3 für eine lineare oder verzweigte gesättigte Alkylgruppe mit 6 bis 30, vorzugsweise 8 bis 22 und weiter bevorzugt 12 bis 18 Kohlenstoffatomen oder eine lineare oder verzweigte ein- oder mehrfach ungesättigte Alkenylgruppe mit 6 bis 30, vorzugsweise 8 bis 22 und weiter bevorzugt 12 bis 18 Kohlenstoffatomen steht und in dem Fall, dass R1 für COOR3 steht und R2 und R4 für H stehen, R3 auch für Phenyl oder Benzyl und vorzugsweise auch für Benzyl stehen kann.

**10.** Alkoxylat nach einem oder mehreren der Ansprüche 1 bis 8, **dadurch gekennzeichnet, dass** R3 für eine lineare oder verzweigte gesättigte Alkylgruppe mit 6 bis 30, vorzugsweise 8 bis 22 und weiter bevorzugt 12 bis 18 Kohlenstoffatomen, eine lineare oder verzweigte ein- oder mehrfach ungesättigte Alkenylgruppe mit 6 bis 30, vorzugsweise 8 bis 22 und weiter bevorzugt 12 bis 18 Kohlenstoffatomen oder $(CH_2CH_2O)_m$Phenyl, wobei m im molaren Durchschnitt für eine Zahl von 1 bis 100, vorzugsweise von 1 bis 10, weiter bevorzugt von 1 bis 3 und noch weiter bevorzugt von 1 bis 2 steht, steht.

**11.** Alkoxylat nach einem oder mehreren der Ansprüche 1 bis 8 und 10, **dadurch gekennzeichnet, dass** R3 für $(CH_2CH_2O)_m$Phenyl, wobei m im molaren Durchschnitt für eine Zahl von 1 bis 10, vorzugsweise von 1 bis 3 und weiter bevorzugt von 1 bis 2 steht, steht.

**12.** Alkoxylat nach einem oder mehreren der Ansprüche 1 bis 10, **dadurch gekennzeichnet, dass** R3 für eine lineare oder verzweigte gesättigte Alkylgruppe mit 6 bis 30, vorzugsweise 8 bis 22 und weiter bevorzugt 12 bis 18 Kohlenstoffatomen oder eine lineare oder verzweigte ein- oder mehrfach ungesättigte Alkenylgruppe mit 6 bis 30, vorzugsweise 8 bis 22 und weiter bevorzugt 12 bis 18 Kohlenstoffatomen steht.

**13.** Alkoxylat nach einem oder mehreren der Ansprüche 1 bis 10 und 12, **dadurch gekennzeichnet, dass** R3 aus der Gruppe bestehend aus n-Hexyl, 3-Methyl-3-pentyl, n-Heptyl, n-Octyl, n-Nonyl, n-Decyl, n-Undecyl, Lauryl, n-Tridecyl, Isotridecyl, Myristyl, n-Pentadecyl, Cetyl, Palmitoleyl, n-Heptadecyl, Stearyl, Oleyl, n-Nonadecyl, Arachidyl, Heneicosyl, Behenyl, Erucyl, n-Tetracosanyl, Ceryl, 1-Heptacosanyl, n-Octacosanyl, n-Nonacosanyl und Myricyl ausgewählt ist.

**14.** Alkoxylat nach einem oder mehreren der Ansprüche 1 bis 10, 12 und 13, **dadurch gekennzeichnet, dass** R3 aus der Gruppe bestehend aus Stearyl, Oleyl und Isotridecyl und vorzugsweise aus der Gruppe bestehend aus Oleyl und Isotridecyl ausgewählt ist.

**15.** Alkoxylat nach einem oder mehreren der Ansprüche 1 bis 10 und 12 bis 14, **dadurch gekennzeichnet, dass**

a) R1 und R4 für H stehen, R2 für COOR3 steht, R3 für Oleyl steht, X für Ethoxy steht, T für H steht und u im molaren Durchschnitt für eine Zahl von 8 bis 28 steht oder
b) R1 und R4 für H stehen, R2 für COOR3 steht, R3 für Isotridecyl steht, X für Ethoxy steht, T für H steht und u im molaren Durchschnitt für eine Zahl von 8 bis 16 steht oder
c) R1 und R4 für H stehen, R2 für COOR3 steht, R3 für Isotridecyl steht, X für Ethoxy steht, T für H steht und u im molaren Durchschnitt für eine Zahl von 17 bis 28 steht oder
d) R1 für COOR3 steht, R2 und T4 für H stehen, R3 für Oleyl steht, X für Ethoxy steht, T für H steht und u im molaren Durchschnitt für eine Zahl von 6 bis 12 steht.

**16.** Alkoxylat nach einem oder mehreren der Ansprüche 1 bis 15, **dadurch gekennzeichnet, dass** es sich um ein Gemisch von zwei oder mehr Verbindungen gemäß Formel (I) handelt.

**17.** Verfahren zur Herstellung eines Alkoxylats nach einem oder mehreren der Ansprüche 1 bis 16, **dadurch gekennzeichnet, dass** einer der Substituenten R1, R2 oder R4 in Formel (I) für COO-$[X_1]_v$-R3 steht und dass in einem ersten Schritt ortho-, meta- oder para-Hydroxybenzoesäure verestert wird oder ein ortho-, meta- oder para-Hydroxybenzoesäureester eines Alkohols mit 1 bis 4 Kohlenstoffatomen umgeestert wird, und zwar jeweils mit einem Alkohol HO-$[X_1]_v$-R3, wobei $X_1$, v und R3 die in einem oder mehreren der Ansprüche 1 bis 16 angegebene Bedeutung haben, bei einer Temperatur von 140 bis 250 °C in Gegenwart eines Katalysators, und in einem zweiten Schritt der im ersten Schritt erhaltene Ester in Gegenwart eines Katalysators bei einem Druck von 1 bis 100 bar und einer Temperatur von 75 bis 220 °C mit Ethylenoxid und/oder Propylenoxid umgesetzt wird und in einem fakultativen dritten Schritt das Umsetzungsprodukt aus dem zweiten Schritt mit einem eine $C_1$-$C_4$-Alkylgruppe liefernden Alkylierungsmittel, mit einem Carboxymethylierungsmittel, mit einem Sulfatierungsmittel, mit einem Phosphatierungsmittel oder mit einem Sulfosuccinierungsmittel umgesetzt wird.

**18.** Verfahren zur Herstellung eines Alkoxylats nach einem oder mehreren der Ansprüche 1, 2, 5, 9 bis 14 und 16, **dadurch gekennzeichnet, dass** einer der Substituenten R1, R2 oder R4 in Formel (I) für CO-NR8R3 steht und dass in einem ersten Schritt ortho-, meta- oder para-Hydroxybenzoesäurechlorid oder ortho-, meta- oder para-Hydroxybenzoesäuremethylester mit einem Amin HNR8R3, wobei R8 und R3 die in einem oder mehreren der Ansprüche 1, 2, 5, 9 bis 14 und 16 angegebene Bedeutung haben, umgesetzt wird und in einem zweiten Schritt das im ersten Schritt erhaltene Amid in Gegenwart eines Katalysators bei einem Druck von 1 bis 100 bar und einer Temperatur von 75 bis 220 °C mit Ethylenoxid und/oder Propylenoxid umgesetzt wird und in einem fakultativen dritten Schritt das Umsetzungsprodukt aus dem zweiten Schritt mit einem eine $C_1$-$C_4$-Alkylgruppe liefernden Alkylierungsmittel, mit einem Carboxymethylierungsmittel, mit einem Sulfatierungsmittel, mit einem Phosphatierungsmittel oder mit einem Sulfosuccinierungsmittel umgesetzt wird.

## Revendications

**1.** Alcoxylate selon la formule suivante (I)

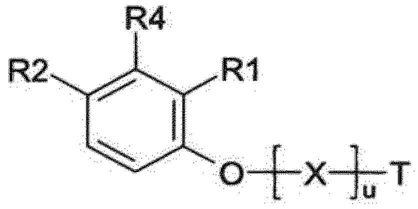

(I)

dans lequel

X est choisi parmi éthoxy et des mélanges de groupes éthoxy et propoxy, est de préférence choisi parmi éthoxy et des mélanges de groupes éthoxy et propoxy où le nombre de groupes éthoxy dans les mélanges est supérieur au nombre de groupes propoxy et, plus préférablement, X est éthoxy,
T est choisi dans le groupe constitué de H, alkyle en $C_1$-$C_4$, $SO_3^-$, $CH_2$-$COO^-$, sulfosuccinate et $PO_3^{2-}$, est de préférence choisi dans le groupe constitué de H et $CH_3$ et, plus préférablement, est H,
R1, R2 et R4 sont H, CO-NR8R3 ou COO-$[X_1]_v$-R3, où deux des substituants R1, R2 et R4 sont H et le troisième de ces substituants est CO-NR8R3 ou COO-$[X_1]_v$-R3 et, de préférence, est COO-$[X_1]_v$-R3,
R8 est H ou un groupe alkyle linéaire ou ramifié avec 1 à 4 atomes C, de préférence H ou $CH_3$ et, plus préférablement, H,
R3 est un groupe alkyle saturé linéaire ou ramifié avec 6 à 30, de préférence 8 à 22 et, plus préférablement, 12 à 18 atomes de carbone, un groupe alcényle mono- ou polyinsaturé linéaire ou ramifié avec 6 à 30, de préférence 8 à 22 et, plus préférablement, 12 à 18 atomes de carbone, un groupe aryle avec 6 à 10 atomes de carbone, de préférence phényle, un groupe $(CH_2CH_2O)_m$aryle, où le groupe aryle comprend 6 à 10 atomes de carbone et m, en moyenne molaire, est un nombre de 1 à 100, de préférence de 1 à 10, plus préférablement de 1 à 3 et encore plus préférablement de 1 à 2, et, de préférence, $(CH_2CH_2O)_m$aryle est $(CH_2CH_2O)_m$phényle, ou un groupe alkyle en $C_1$-$C_3$ linéaire ou ramifié substitué par aryle, où le groupe aryle comprend 6 à 10 atomes de carbone, de préférence benzyle, mais dans le cas où R1 et R4 sont H et R2 est COOR3, R3 peut uniquement être un groupe alkyle, un groupe alcényle ou un groupe $(CH_2CH_2O)_m$aryle tel que défini ci-dessus et dans ce cas, R3 est de préférence un groupe alkyle ou un groupe alcényle tel que défini ci-dessus,
$X_1$ est choisi parmi éthoxy et des mélanges de groupes éthoxy et propoxy, est de préférence choisi parmi éthoxy et des mélanges de groupes éthoxy et propoxy où le nombre de groupes éthoxy dans les mélanges est supérieur au nombre de groupes propoxy et, plus préférablement, $X_1$ est éthoxy,
u, en moyenne molaire, est un nombre de 3 à 100, de préférence de 5 à 60, plus préférablement de 6 à 50, encore plus préférablement de 8 à 40 et, de façon particulièrement préférable, de 9 à 35, et
v, en moyenne molaire, est un nombre de 0 à 20, de préférence de 0 à 12, plus préférablement de 0 à 10, encore plus préférablement de 0 à 8 et, de façon particulièrement préférable, de 0 à 7.

**2.** Alcoxylate selon la revendication 1, **caractérisé en ce que**
X est choisi parmi éthoxy et des mélanges de groupes éthoxy et propoxy, est de préférence choisi parmi éthoxy et des mélanges de groupes éthoxy et propoxy où le nombre de groupes éthoxy dans les mélanges est supérieur au

nombre de groupes propoxy et, plus préférablement, X est éthoxy,
T est choisi dans le groupe constitué de H, alkyle en $C_1$-$C_4$, $SO_3^-$, $CH_2$-$COO^-$, sulfosuccinate et $PO_3^{2-}$, est de préférence choisi dans le groupe constitué de H et $CH_3$ et, plus préférablement, est H,
R1, R2 et R4 sont H, CO-NR8R3 ou COO-$[X_1]_v$-R3, où deux des substituants R1, R2 et R4 sont H et le troisième de ces substituants est CO-NR8R3 ou COO-$[X_1]_v$-R3 et, de préférence, est COO-$[X_1]_v$-R3,
R8 est H ou un groupe alkyle linéaire ou ramifié avec 1 à 4 atomes C, de préférence H ou $CH_3$ et, plus préférablement, H,
R3 est un groupe alkyle saturé linéaire ou ramifié avec 6 à 30, de préférence 8 à 22 et, plus préférablement, 12 à 18 atomes de carbone, un groupe alcényle mono- ou polyinsaturé linéaire ou ramifié avec 6 à 30, de préférence 8 à 22 et, plus préférablement, 12 à 18 atomes de carbone, ou $(CH_2CH_2O)_m$phényle, dans lequel m, en moyenne molaire, est un nombre de 1 à 100, de préférence de 1 à 10, plus préférablement de 1 à 3 et encore plus préférablement de 1 à 2, et dans le cas où R1 ou R4 est COO-$[X_1]_v$-R3 et les autres substituants de R1, R2 et R4 sont H et, de préférence, dans le cas où R1 est COO-$[X_1]_v$-R3 et R2 et R4 sont H, R3 peut également être phényle ou benzyle et, de préférence, peut également être benzyle, $X_1$ est choisi parmi éthoxy et des mélanges de groupes éthoxy et propoxy, est de préférence choisi parmi éthoxy et des mélanges de groupes éthoxy et propoxy où le nombre de groupes éthoxy dans les mélanges est supérieur au nombre de groupes propoxy et, plus préférablement, $X_1$ est éthoxy,
u, en moyenne molaire, est un nombre de 3 à 100, de préférence de 5 à 60, plus préférablement de 6 à 50, encore plus préférablement de 8 à 40 et, de façon particulièrement préférable, de 9 à 35, et
v, en moyenne molaire, est un nombre de 0 à 20, de préférence de 0 à 12, plus préférablement de 0 à 10, encore plus préférablement de 0 à 8 et, de façon particulièrement préférable, de 0 à 7.

3. Alcoxylate selon la revendication 1 ou 2, **caractérisé en ce que** deux des substituants R1, R2 et R4 sont H et le troisième de ces substituants est COO-$[X_1]_v$-R3 et la somme u + v, en moyenne molaire, est un nombre de 3 à 100, de préférence de 5 à 60, plus préférablement de 6 à 50, encore plus préférablement de 8 à 40 et, de façon particulièrement préférable, de 9 à 35.

4. Alcoxylate selon une ou plusieurs des revendications 1 à 3, **caractérisé en ce que** deux des substituants R1, R2 et R4 sont H et the troisième de ces substituants est COOR3.

5. Alcoxylate selon une ou plusieurs des revendications 1 à 4, **caractérisé en ce que** R4 est H.

6. Alcoxylate selon une ou plusieurs des revendications 1 à 5, **caractérisé en ce que**
X est choisi parmi éthoxy et des mélanges de groupes éthoxy et propoxy, est de préférence choisi parmi éthoxy et des mélanges de groupes éthoxy et propoxy où le nombre de groupes éthoxy dans les mélanges est supérieur au nombre de groupes propoxy et, plus préférablement, X est éthoxy,
T est H,
R1 et R2 sont H ou COOR3, où un des substituants R1 et R2 est H et l'autre de ces substituants est COOR3,
R3 est un groupe alkyle saturé linéaire ou ramifié avec 6 à 30, de préférence 8 à 22 et, plus préférablement, 12 à 18 atomes de carbone, un groupe alcényle mono- ou polyinsaturé linéaire ou ramifié avec 6 à 30, de préférence 8 à 22 et, plus préférablement, 12 à 18 atomes de carbone ou $(CH_2CH_2O)_m$phényle, où m, en moyenne molaire, est un nombre de 1 à 100, de préférence de 1 à 10, plus préférablement de 1 à 3 et encore plus préférablement de 1 à 2, et dans le cas où R1 est COOR3 et R2 est H, R3 peut également être phényle ou benzyle et, de préférence, peut également être benzyle,
R4 est H, et
u, en moyenne molaire, est un nombre de 3 à 100, de préférence de 5 à 60, plus préférablement de 6 à 50, encore plus préférablement de 8 à 40 et, de façon particulièrement préférable, de 9 à 35.

7. Alcoxylate selon une ou plusieurs des revendications 1 à 6, **caractérisé en ce que** R1 et R4 sont H et R2 est COOR3.

8. Alcoxylate selon une ou plusieurs des revendications 1 à 6, **caractérisé en ce que** R1 est COOR3 et R2 et R4 sont H.

9. Alcoxylate selon une ou plusieurs des revendications 1 à 8, **caractérisé en ce que** R3 est un groupe alkyle saturé linéaire ou ramifié avec 6 à 30, de préférence 8 à 22 et, plus préférablement, 12 à 18 atomes de carbone ou un groupe alcényle mono- ou polyinsaturé linéaire ou ramifié avec 6 à 30, de préférence 8 à 22 et, plus préférablement, 12 à 18 atomes de carbone et dans le cas où R1 est COOR3 et R2 et R4 sont H, R3 peut également être phényle ou benzyle et, de préférence, peut également être benzyle.

**10.** Alcoxylate selon une ou plusieurs des revendications 1 à 8, **caractérisé en ce que** R3 est un groupe alkyle saturé linéaire ou ramifié avec 6 à 30, de préférence 8 à 22 et, plus préférablement, 12 à 18 atomes de carbone ou un groupe alcényle mono- ou polyinsaturé linéaire ou ramifié avec 6 à 30, de préférence 8 à 22 et, plus préférablement, 12 à 18 atomes de carbone ou $(CH_2CH_2O)_m$phényle, où m, en moyenne molaire, est un nombre de 1 à 100, de préférence de 1 à 10, plus préférablement de 1 à 3 et encore plus préférablement de 1 à 2.

**11.** Alcoxylate selon une ou plusieurs des revendications 1 à 8 et 10, **caractérisé en ce que** R3 est $(CH_2CH_2O)_m$phényle, où m, en moyenne molaire, est un nombre de 1 à 10, de préférence de 1 à 3 et, plus préférablement, de 1 à 2.

**12.** Alcoxylate selon une ou plusieurs des revendications 1 à 10, **caractérisé en ce que** R3 est un groupe alkyle saturé linéaire ou ramifié avec 6 à 30, de préférence 8 à 22 et, plus préférablement, 12 à 18 atomes de carbone ou un groupe alcényle mono- ou polyinsaturé linéaire ou ramifié avec 6 à 30, de préférence 8 à 22 et, plus préférablement, 12 à 18 atomes de carbone.

**13.** Alcoxylate selon une ou plusieurs des revendications 1 à 10 et 12, **caractérisé en ce que** R3 est choisi dans le groupe constitué de n-hexyle, 3-méthyl-3-pentyle, n-heptyle, n-octyle, n-nonyle, n-décyle, n-undécyle, lauryle, n-tridécyle, isotridécyle, myristyle, n-pentadécyle, cétyle, palmitoléyle, n-heptadécyle, stéaryle, oléyle, n-nonadécyle, arachidyle, héneicosyle, béhényle, érucyle, n-tétracosanyle, céryle, 1-heptacosanyle, n-octacosanyle, n-nonacosanyle et myricyle.

**14.** Alcoxylate selon une ou plusieurs des revendications 1 à 10, 12 et 13, **caractérisé en ce que** R3 est choisi dans le groupe constitué de stéaryle, oléyle et isotridécyle et est de préférence choisi dans le groupe constitué d'oléyle et isotridécyle.

**15.** Alcoxylate selon une ou plusieurs des revendications 1 à 10 et 12 à 14, **caractérisé en ce que**

a) R1 et R4 sont H, R2 est COOR3, R3 est oléyle, X est éthoxy, T est H et u, en moyenne molaire, est un nombre de 8 à 28, ou
b) R1 et R4 sont H, R2 est COOR3, R3 est isotridécyle, X est éthoxy, T est H et u, en moyenne molaire, est un nombre de 8 à 16, ou
c) R1 et R4 sont H, R2 est COOR3, R3 est isotridécyle, X est éthoxy, T est H et u, en moyenne molaire, est un nombre de 17 à 28, ou
d) R1 est COOR3, R2 et R4 sont H, R3 est oléyle, X est éthoxy, T est H et u, en moyenne molaire, est un nombre de 6 à 12.

**16.** Alcoxylate selon une ou plusieurs des revendications 1 à 15, **caractérisé en ce qu'**il est un mélange de deux ou plus de deux composés selon la formule (I).

**17.** Procédé de préparation d'un alcoxylate selon une ou plusieurs des revendications 1 à 16, **caractérisé en ce que** l'un des substituants R1, R2 ou R4 dans la formule (I) est COO-$[X_1]_v$-R3 et que, dans une première étape, l'acide ortho-, méta- ou para-hydroxybenzoïque est estérifié ou un ester d'acide ortho-, méta- ou para-hydroxybenzoïque d'un alcool avec 1 à 4 atomes de carbone est transestérifié, dans chaque cas avec un alcool HO-$[X_1]_v$-R3, où $X_1$, v et R3 ont la signification décrite dans une ou plusieurs des revendications 1 à 16 à une température de 140 à 250 °C en présence d'un catalyseur et, dans une deuxième étape, l'ester obtenu dans la première étape réagit avec l'oxyde d'éthylène et/ou l'oxyde de propylène en présence d'un catalyseur à une pression de 1 à 100 bar et à une température de 75 à 220 °C et, dans une troisième étape facultative, le produit de réaction de la deuxième étape réagit avec un agent d'alkylation fournissant un groupe alkyle en $C_1$-$C_4$, avec un agent de carboxyméthylation, avec un agent de sulfatation, avec un agent de phosphatation ou avec un agent de sulfosuccinatation.

**18.** Procédé de préparation d'un alcoxylate selon une ou plusieurs des revendications 1, 2, 5, 9 à 14 et 16, **caractérisé en ce que** l'un des substituants R1, R2 ou R4 dans la formule (I) est CO-NR8R3 et que, dans une première étape, le chlorure d'acide ortho-, méta- ou para-hydroxybenzoïque ou l'ester méthylique d'acide ortho-, méta- ou para-hydroxybenzoïque réagit avec une amine HNR8R3, où R3 et R8 ont la signification décrite dans une ou plusieurs des revendications 1, 2, 5, 9 à 14 et 16 et, dans une deuxième étape, l'amide obtenue dans la première étape réagit avec l'oxyde d'éthylène et/ou l'oxyde de propylène en présence d'un catalyseur à une pression de 1 à 100 bar et à une température de 75 à 220 °C et, dans une troisième étape facultative, le produit de réaction de la deuxième étape réagit avec un agent d'alkylation fournissant un groupe alkyle en $C_1$-$C_4$, avec un agent de carboxyméthylation, avec un agent de sulfatation, avec un agent de phosphatation ou avec un agent de sulfosuccinatation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4240918 E
- US 4548744 A
- JP 2009256214 A
- US 5480761 A
- EP 2703347 A
- WO 2008138486 A1


### Non-patent literature cited in the description


- **R. STEVANOVA ; D. RANKOFF ; S. PANAYOTOVA ; S.L. SPASSOV.** *J. Am. Oil Chem. Soc.,* 1988, vol. 65, 1516-1518